(19) **Europäisches Patentamt / European Patent Office / Office européen des brevets**

(11) **EP 4 092 130 A1**

(12) **EUROPEAN PATENT APPLICATION**
published in accordance with Art. 153(4) EPC

(43) Date of publication:
**23.11.2022 Bulletin 2022/47**

(21) Application number: **20913622.5**

(22) Date of filing: **17.01.2020**

(51) International Patent Classification (IPC):
**C12Q 1/68** $^{(2018.01)}$      **C12M 1/00** $^{(2006.01)}$

(52) Cooperative Patent Classification (CPC):
**C12M 1/00; C12Q 1/68**

(86) International application number:
**PCT/CN2020/072841**

(87) International publication number:
**WO 2021/142794 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BGI Shenzhen**
**Shenzhen, Guangdong 518083 (CN)**

(72) Inventors:
• **CHEN, Ruoyan**
  **Shenzhen, Guangdong 518083 (CN)**
• **GAO, Ya**
  **Shenzhen, Guangdong 518083 (CN)**

• **XU, Jinjin**
  **Shenzhen, Guangdong 518083 (CN)**
• **SU, Fengxia**
  **Shenzhen, Guangdong 518083 (CN)**
• **LIN, Yu**
  **Shenzhen, Guangdong 518083 (CN)**
• **JIN, Xin**
  **Shenzhen, Guangdong 518083 (CN)**
• **CHEN, Fang**
  **Shenzhen, Guangdong 518083 (CN)**

(74) Representative: **Habermann, Hruschka & Schnabel**
**Patentanwälte**
**Montgelasstraße 2**
**81679 München (DE)**

(54) **METHOD FOR DETERMINING FOETAL NUCLEIC ACID CONCENTRATION AND FOETAL GENOTYPING METHOD**

(57)    Provided in the present disclosure are a method for determining fetal nucleic acid concentration and a fetal genotyping method. According to the embodiments of the present disclosure, the method for determining cell-free fetal nucleic acid concentration includes: (1) acquiring sequencing data of a first nucleic acid sample of a pregnant woman and a reference genome sequence, the first nucleic acid sample of the pregnant woman containing cell-free fetal nucleic acids, and the sequencing data being composed of a plurality of sequencing reads; (2) selecting a predetermined region on the reference genome sequence and determining, based on the sequencing data of the first nucleic acid sample of the pregnant woman, mutation information in the predetermined region; and (3) determining the concentration of cell-free fetal nucleic acids corresponding to the predetermined region based on the mutation information in the predetermined region.

EP 4 092 130 A1

**Description**

**TECHNICAL FIELD**

The present disclosure relates to the field of biotechnology, in particular to non-invasive prenatal genetic testing, and specifically to a method for determining fetal nucleic acid concentration and a fetal genotyping method.

**BACKGROUND**

Prenatal diagnosis methods are generally classified into two categories, i.e., invasive methods and non-invasive methods, depending on the material acquiring and examination methods. The former mainly includes amniocentesis, chorionic villus sampling (CVS), umbilical cord blood sampling, etc.; the latter includes ultrasonography, maternal peripheral blood serum marker determination, and fetal cell detection, etc. Cells isolated from the fetus by invasive procedures such as CVS or amniocentesis can be used for routine prenatal diagnosis. While this method is highly accurate for the diagnosis of fetal aneuploidy, these conventional methods are invasive and pose a risk to the mother and fetus.

At present, the methods for fetal genome inference using maternal plasma cfDNA and parental WGS data require simultaneous collection of blood samples from father and mother, and WGS sequencing of maternal plasma cfDNA and blood cells of father and mother. The sampling is difficult, and the costs of sequencing and calculation are high. Meanwhile, if this method is used to complete the detection of fetal de novo mutations, it is necessary to further increase the cost to achieve limit sequencing of the maternal plasma, with a very low detection specificity. Although the method of fetal genome inference based on maternal plasma cfDNA data and single cell sequencing data only requires to sample maternal whole blood, both maternal plasma cfDNA sequencing and single cell sequencing of lymphocytes are required for fetal genotype inference over the whole genome, resulting in excessively high experimental requirements and very high cost for haplotype inference of father and mother. Fetal de novo mutation detection is unavailable with this method. In addition, since only a fixed threshold is used to determine fetal genotype for each locus, the method of fetal genome inference only based on maternal plasma cfDNA ultra-high-depth sequencing data requires extremely high sequencing depth (300X or higher), and fetal genotype cannot be accurately inferred when sequencing depth is low (within 100X) or the fetal fraction is low.

However, the methods for non-invasive diagnosis of fetuses remain to be improved at present.

**SUMMARY**

The present disclosure aims to solve at least one of the technical problems existing in the related art. Therefore, an object of the present disclosure is to propose a method for accurately and efficiently determining chromosomal aneuploidy.

In a first aspect, the present disclosure provides a method for determining the concentration of cell-free fetal nucleic acids. According to embodiments of the present disclosure, the method includes: (1) acquiring sequencing data of a first nucleic acid sample of a pregnant woman and a reference genome sequence, the first nucleic acid sample of the pregnant woman containing cell-free fetal nucleic acids, and the sequencing data being composed of a plurality of sequencing reads; (2) selecting a predetermined region on the reference genome sequence, and determining, based on the sequencing data of the first nucleic acid sample of the pregnant woman, mutation information in the predetermined region; and (3) determining the concentration of cell-free fetal nucleic acids corresponding to the predetermined region based on the mutation information in the predetermined region. According to the embodiments of the present disclosure, by determining the distribution of mutations in a predetermined region, the fetal nucleic acid concentration corresponding to the region can be effectively determined, and then can be effectively applied to the in-depth analysis of cell-free fetal nucleic acids. Furthermore, according to the embodiments of the present disclosure, it has been found that the method can be suitable for sequencing results with a relatively low depth, e.g., a sequencing depth smaller than or equal to 100X, e.g., smaller than or equal to 60X, and also suitable for samples having a relatively low fetal fraction, e.g., a fetal fraction smaller than or equal to 10%, and thus can be effectively applied to the early prenatal diagnosis of a pregnant woman, e.g., 15 weeks of gestation.

In a second aspect of the disclosure, the present disclosure provides a method for determining a fetal genotype at a predetermined locus. According to embodiments of the present disclosure, the method includes: (a) determining a concentration of cell-free fetal nucleic acids in the predetermined region including the predetermined locus, according to the method described above; (b) determining a number $A_j$ (j being A, T, G, or C) of sequencing reads supporting base A, base T, base G, or base C, respectively, for the predetermined locus; (c) constructing a genotype set $\{M_{1i}M_{2i}P_{1i}P_{2i}\}$ for the predetermined locus, wherein i denotes a genotype serial number, $M_{1i}$ denotes a base type of the predetermined locus on a first maternal chromosome for the i-th genotype, $M_{2i}$ denotes a base type of the predetermined locus on a second maternal chromosome for the i-th genotype, $F_{1i}$ denotes a base type of the predetermined locus on a first fetal chromosome for the i-th genotype, $F_{2i}$ denotes a base type of the predetermined locus on a second fetal chromosome for the i-th genotype, and $M_{1i}$, $M_{2i}$, $F_{1i}$, and $F_{2i}$ are each independently base A, base T, base G, or base C, wherein the first maternal chromosome and the second maternal chromosome constitute a pair of homologous chromosomes, and the first fetal chromosome and the second fetal chromosome constitute a pair of homologous chromosomes; (d) determining a probability $P_j$ of occurrence of each base for each genotype of the genotype set $\{M_{1i}M_{2i}P_{1i}P_{2i}\}$ based on the cell-free fetal nucleic acid concentration, wherein j is A, T, G, or C; (e) determining a cumulative probability $P (M_{1i}M_{2i}P_{1i}P_{2i})$ of each genotype of the genotype set $\{M_{1i}M_{2i}P_{1i}P_{2i}\}$ based on the probability Pj of occurrence of each base and the number $A_j$ of the sequencing reads; and (f) determining a combination of a maternal genotype and a fetal genotype at the predetermined locus based on the cumulative probability $P (M_{1i}M_{2i}P_{1i}P_{2i})$ of each genotype, thereby obtaining the fetal genotype at the predetermined locus. In this way, the combination of the fetal genotype and the maternal genotype with the highest probability can be determined effectively based on the fetal nucleic acid concentration in the predetermined region as well as the number of sequencing reads sequenced. In other words, a haplotype at a specific locus in the region can be determined.

In a third aspect, the present disclosure provides a device for determining a concentration of cell-free fetal nucleic acids. According to embodiments of the present disclosure, the device includes: a reading module configured to acquire sequencing data of a first nucleic acid sample of a pregnant woman and a reference genome sequence, the first nucleic acid sample of the pregnant woman containing cell-free fetal nucleic acids, and the sequencing data being composed of a plurality of sequencing reads; a mutation determination module configured to select a predetermined region on the reference genome sequence and determining, based on the sequencing data of the first nucleic acid sample of the pregnant woman, mutation information in the predetermined region; and a cell-free fetal nucleic acid concentration determination module configured to determine the concentration of cell-free fetal nucleic acids corresponding to the predetermined region based on the mutation information in the predetermined region. According to the embodiment of the present disclosure, the device is capable of effectively implementing the foregoing method for determining the concentration of cell-free fetal nucleic acids . The advantages and features described with respect to the above method are applicable to the device and will not be described in detail.

In a fourth aspect, the present disclosure provides an apparatus for determining a fetal genotype at a predetermined locus. The apparatus includes: the device for determining the concentration of cell-free fetal nucleic acids as mentioned above for determining the concentration of cell-free fetal nucleic acids in the predetermined region, the predetermined region including the predetermined locus; a sequencing read number determination module configured to determine a number $A_j$ (j being A, T, G, or C) of sequencing reads supporting base A, base T, base G, or base C, respectively, for the predetermined locus; a genotype set construction module configured to construct a genotype set $\{M_{1i}M_{2i}P_{1i}P_{2i}\}$ for the predetermined locus, wherein i denotes a genotype serial number, $M_{1i}$ denotes a base type of the predetermined locus on a first maternal chromosome for the i-th genotype, $M_{2i}$ denotes a base type of the predetermined locus on a second maternal chromosome for the i-th genotype, Fii denotes a base type of the predetermined locus on a first fetal chromosome for the i-th genotype, $F_{2i}$ denotes a base type of the predetermined locus on a second fetal chromosome for the i-th genotype, and $M_{1i}$, $M_{2i}$, $F_{1i}$, and $F_{2i}$ are each independently base A, base T, base G, or base C, wherein the first maternal chromosome and the second maternal chromosome constitute a pair of homologous chromosomes, and the first fetal chromosome and the second fetal chromosome constitute a pair of homologous chromosomes; an occurrence probability determination module configured to determine a probability $P_j$ of occurrence of each base for each genotype of the genotype set $\{M_{1i}M_{2i}P_{1i}P_{2i}\}$ based on the concentration of cell-free fetal nucleic acids, wherein j is A, T, G, or C; a cumulative probability determination module configured to determine a cumulative probability $P (M_{1i}M_{2i}P_{1i}P_{2i})$ of each genotype of the genotype set $\{M_{1i}M_{2i}P_{1i}P_{2i}\}$ based on the probability Pj of occurrence of each base and the number $A_j$ of the sequencing reads; and a genotype combination determination module configured to determine a combination of a maternal genotype and a fetal genotype at the predetermined locus based on the cumulative probability $P (M_{1i}M_{2i}P_{1i}P_{2i})$ of each genotype, thereby obtaining the fetal genotype at the predetermined locus.

In a fifth aspect of the present disclosure, the present disclosure provides a computer-readable storage medium having a computer program stored thereon. The program, when executed by a processor, implements the steps of the method described above.

The advantages of the present disclosure are as follows:

(1) At present, most fetal genome analysis based on maternal plasma cfDNA whole genome sequencing data focuses on the detection of chromosomal level structural variation, large fragment copy number mutation, and paternal specific variation; the present disclosure can extend the region of fetal genome analysis to whole genome, improve the detection accuracy to single base mutation, and expand the application range of fetal genetic disease detection.

(2) Most of the existing fetal whole genome analysis methods need the assistance of parental blood cell WGS data; the present disclosure provides multiple analysis schemes depending on parental WGS data or independent of parental WGS data, providing diversity methodological support for different types of samples and data.

(3) Fetal genome analysis methods currently performed using only maternal plasma cfDNA have very high requirements for sequencing depth (300X or above), while the present disclosure still achieves high accuracy at relatively low sequencing depths (about 60X to 100X).

Additional aspects and advantages of the present disclosure will be set forth in part in the description which follows and, in part become apparent from the description, or may be learned by practice of the present disclosure.

## BRIEF DESCRIPTION OF DRAWINGS

The foregoing and/or additional aspects and advantages of the present disclosure will become apparent and readily appreciated from the following description of the embodiments taken in conjunction with the accompanying drawings, in which:

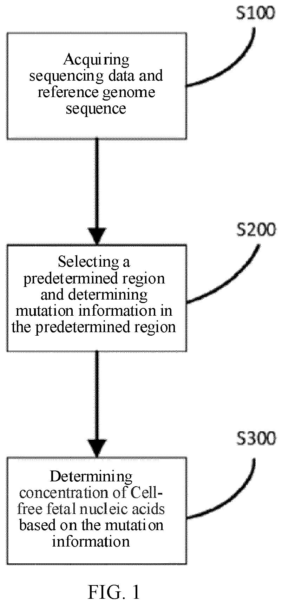

FIG. 1 is a schematic flowchart of a method for determining a fetal nucleic acid concentration according to an embodiment of the present disclosure;
FIG. 2 is a schematic flowchart of a method for determining a fetal genotype at a predetermined locus according to an embodiment of the present disclosure;
FIG. 3 is a schematic flowchart of a method for determining a fetal genotype at a predetermined locus according to another embodiment of the present disclosure;
FIG. 4 is a schematic flowchart of a method for determining a fetal genotype at a predetermined locus according to an embodiment of the present disclosure;
FIG. 5 is a schematic flowchart of a method for determining a fetal genotype at a predetermined locus according to another embodiment of the present disclosure;
FIG. 6 is a structural representation of a device for determining a concentration of cell-free fetal nucleic acids according to an embodiment of the present disclosure;
FIG. 7 is a structural representation of an apparatus for determining a fetal genotype at a predetermined locus according to an embodiment of the present disclosure;
FIG. 8 is a structural representation of an apparatus for determining a fetal genotype at a predetermined locus according to another embodiment of the present disclosure;
FIG. 9 is a structural representation of an apparatus for determining a fetal genotype at a predetermined locus according to another embodiment of the present disclosure;
FIG. 10 is a structural representation of an apparatus for determining a fetal genotype at a predetermined locus according to an embodiment of the present disclosure;
FIG. 11 is a structural representation of an apparatus for determining a fetal genotype at a predetermined locus according to an embodiment of the present disclosure;
FIG. 12 shows a schematic diagram of a relationship between a minor allele frequency and a distribution of a proportion of loci;
FIG. 13 shows a schematic diagram of schemes 1 to 4 according to embodiments of the present disclosure; and
FIG. 14 shows a comparison of accuracy of Scheme 4 *vs*. Scheme 3 after correction of the maternal-heterozygous-and-fetal-heterozygous locus in Family 1 is completed by setting different numbers of flanking loci in Example 1 of the present disclosure.

## DESCRIPTION OF EMBODIMENTS

The embodiments of the present disclosure will be described in detail below. The embodiments described below are exemplary, and are only used to explain the present disclosure but should not be construed as a limitation to the present disclosure. The embodiments of the present disclosure will be described in detail below. The embodiments described below are exemplary, and are only used to explain the present disclosure but should not be construed as a limitation to the present disclosure. It should be appreciated that the present disclosure may be applied to various general-purpose or dedicated computing device environments or configurations, for example, a personal computer, a server computer, a handheld device or a portable device, a flat panel device, a multi-processor device, and a distributed computing environment including any one of the foregoing apparatus or devices. This disclosure may be described in a general context of computer executable instructions executed by the computer, such as a program module. Generally, the program module includes a routine, a program, an object, a component, a data structure, and the like that execute a specified task or implement a specified abstract data type. This disclosure may alternatively be practiced in distributed computing environments. In the distributed computing environments, tasks are executed by remote processing devices connected through a communication network. In the distributed computing environments, the program module may be located in local and remote computer storage media including storage devices.

**Method for determining cell-free fetal nucleic acid concentration**

In a first aspect, the present disclosure provides a method for determining a cell-free fetal nucleic acid concentration. According to embodiments of the present disclosure, the method includes the following steps.

At step S100, sequencing data and a reference genome sequence are obtained.

According to an embodiment of the present disclosure, sequencing data of a nucleic acid sample containing cell-free fetal nucleic acids from a pregnant woman and a reference genome sequence are acquired firstly.

According to an embodiment of the present disclosure, maternal samples that can be used include, but are not limited to, maternal peripheral blood. Dennis Lo et al. found cell-free fetal DNAs in maternal plasma and serum, providing a new idea for non-invasive prenatal testing (NIPT). The use of maternal peripheral blood will not cause trauma to the pregnant woman, thus avoiding the risk of miscarriage due to sampling. According to an embodiment of the present disclosure, after maternal samples, such as maternal peripheral blood, are obtained, the samples may be subjected to nucleic acid sequencing in order to obtain nucleic acid sequencing data of the maternal samples, which is typically composed of a plurality or a large number of sequencing reads. According to an embodiment of the present disclosure, the method for sequencing nucleic acid molecules of the maternal samples is not particularly limited, and in particular, any sequencing method known to those skilled in the art may be used, for example including but not limited to sequencing the nucleic acid molecules of the maternal sample by paired-end/mate-pair sequencing, single-read sequencing, or singlemolecule sequencing.

In addition, according to an embodiment of the present disclosure, there is no particular requirement for the sequencing depth for sequencing the maternal sample; according to an embodiment of the present disclosure, the inventors of the present disclosure have found that by analyzing the distribution of mutation types using the method of the present disclosure, sequencing results with a low sequencing depth can also achieve higher accuracy, for example, higher accuracy can also be achieved for sequencing data with a sequencing depth below 100X, in particular for sequencing data with a sequencing depth from 60X to 100X. As will be appreciated by those skilled in the art, after nucleic acid sequencing data is obtained, the resulting sequencing data composed of a large number of sequencing reads can be subjected to filtration and screening treatment according to quality control standards to remove sequencing reads with sequencing quality problems, so that the accuracy of subsequent data analysis can be improved.

According to an embodiment of the present disclosure, the reference genome sequence that can be employed is not particularly limited and may be a part of any known human genome sequence, e.g. available from the sequence published by NCBI (https://www.ncbi.nlm.nih.gov/grc/human). Of course, it will be appreciated by those skilled in the art that the reference genome sequence employed herein may not necessarily require a full length of the human genome sequence, and may even not cover the entire predetermined region, but only cover a sufficient number of mutation loci that may be present in the predetermined region. According to an embodiment of the present disclosure, it is necessary to determine a base type distribution of about 50 to 200 mutation loci in order to determine the fetal nucleic acid concentration of the predetermined sequence. Thus, an obtained reference genome sequence can be used provided that the obtained reference genome sequence is capable of covering the number of mutation loci.

At step S200, a predetermined region is selected and mutation information in the predetermined region is determined.

Upon obtaining the sequencing data and the reference genome sequence, a predetermined region may first be selected on the reference genome sequence for subsequent analysis. The existing methods for determining the cell-free fetal nucleic acid concentration usually aim at the total content of all cell-free fetal nucleic acids, and the resulting cell-free fetal nucleic acid concentration cannot be effectively applied to genotyping. After in-depth analysis combined with practical experience, the inventors of the present disclosure propose to partition the reference genome into a plurality of regions, and analyze the cell-free fetal nucleic acid concentration respectively for each of these regions. Thus, typing of fetal genotypes within these regions can be performed subsequently based on the data obtained for the cell-free fetal nucleic acid concentrations in combination with sequencing alignment results.

According to an embodiment of the present disclosure, the predetermined region is greater than or equal to 50 kb in length. According to an embodiment of the present disclosure, the length of the predetermined region is from 50 kb to 200 kb. According to an embodiment of the present disclosure, the length of the predetermined region is 100 kb. The inventors have found that the number of mutations in a predetermined region of this length satisfies the need to determine the cell-free fetal nucleic acid concentration (herein, " cell-free fetal nucleic acid concentration" is sometimes referred to simply as "fetal fraction", and they are used interchangeably). Thus, the efficiency for determining fetal nucleic acid concentration can be improved. According to an embodiment of the present disclosure, the peaks or troughs of the base type distribution can be determined by analyzing the base-type distribution of the mutation loci, and usually the peaks and troughs are determined by the fetal fraction, so that the cell-free fetal nucleic acid concentration in the region can be determined by analyzing the distribution of the base types. To this end, in order to determine the cell-free fetal nucleic acid concentration, a certain number of mutation loci are required as the analysis basis. According to the research of the inventors, 50 mutation loci can support efficient analysis of the concentration of cell-free fetal nucleic acids in this region, and a predetermined nucleic acid region of not less than 50 kb is used in consideration of a probability of mutation. In addition, considering that the obtained cell-free fetal nucleic acid concentrations are subsequently subjected to fetal genotyping, if the predetermined region is too long, it may be incapable of representing the true fetal nucleic acid proportion of each mutation locus, and thus, according to an embodiment of the present disclosure, the length of the predetermined region is from 50 kb to 200 kb. According to an embodiment of the present disclosure, the length of the predetermined region is 100 kb. The inventors have found that by using a predetermined region of this length, not only the fetal nucleic acid concentration within the region can be effectively determined, but also the fetal nucleic acid concentration can truly reflect the fetal nucleic acid proportion of the mutation locus, and thus can be effectively applied for fetal genotyping within this region.

The term "cell-free fetal nucleic acid concentration" as used herein, also sometimes referred to as "fetal fraction" or "fetal nucleic acid concentration", and these terms were used interchangeably and refer to, for a given genomic region, a proportion of cell-free fetal nucleic acids in the peripheral blood of a pregnant woman corresponding to that region to the total amount of cell-free fetal nucleic acids and cell-free maternal nucleic acids in the peripheral blood of a pregnant woman corresponding to that region. For example, for a given genomic region, there are 100 cell-free nucleic acid molecules corresponding to that region, these 100 cell-free nucleic acid molecules including both cell-free nucleic acid molecules from the pregnant woman and cell-free nucleic acid molecules from the fetus, and if the number of cell-free nucleic acid molecules from the fetus is 30, the cell-free fetal nucleic acid concentration is 30% for that given genomic region.

After the sequencing data and the reference genome sequence are obtained, mutation information in the predetermined region can be determined based on the sequencing data of the pregnant woman. According to an embodiment of the present disclosure, the first nucleic acid sample of the pregnant woman is derived from peripheral blood of the pregnant woman and the mutation information includes at least one of SNP, Indel, or SV, preferably SNP. According to an embodiment of the present disclosure, such information can be easily obtained by sequence alignment, and those skilled in the art can easily obtain the related locus information, for example, obtain through a database a locus known to have a corresponding mutation, so that the corresponding mutation information can be determined for the locus, thereby improving the efficiency of determining the fetal nucleic acid concentration. In addition, according to an embodiment of the present disclosure, the use of peripheral blood of the pregnant woman will not cause trauma to the pregnant woman, thus avoiding the risk of miscarriage due to sampling.

For a specific mutation locus, such as SNP, the base type that can be selected is typically A, T, G, or C. Each of the different base types may have a different proportion, and the inventors of the present disclosure proposed that the base types may be classified into a major allele type and a minor allele type according to the occurrence proportion of each base type. Here, "major allele type" refers to a base type that occurs in the highest proportion for a specific locus, such as a SNP locus. "minor allele type" refers to a base type that occurs in the second highest proportion for a specific locus, such as a SNP locus. Apparently, when the base types occurring at this locus in the pregnant women is different from the base type occurring at this locus in the fetus, both the proportions of the major allele type and the minor allele type are affected by the cell-free fetal nucleic acid concentration, and thus can be used to obtain the cell-free fetal nucleic acid concentration through backward induction.

According to an embodiment of the present disclosure, the numbers of sequencing reads supporting the respective base types can be determined to acquire the proportions of occurrence of the respective base types, or to acquire the frequencies of occurrence of the respective base types in a specific mutation locus. The mutation information to be determined includes mutation loci and the major allele type or minor allele type among the respective base types at each of the mutation loci, and a frequency (proportion of occurrence) of the major allele type or minor allele type. In addition, after the specific base type of each mutation locus, i.e., the major allele type or the minor allele type, is determined, the numbers of mutation loci corresponding to respective frequencies or a proportion of mutation loci corresponding to a respective frequency to all mutation loci in the predetermined region can be further determined. According to an embodiment of the present disclosure, the major allele type or minor allele type and the corresponding frequency are determined by the following steps.

At step S210, the sequencing data is aligned with the reference genome sequence to determine mutation loci in the predetermined region and a base type of each of the mutation loci.

Those skilled in the art would be able to align the sequencing data with the reference genome by conventional means, for example, known software such as SOAP can be used to align the sequencing data with the reference genome sequence. The mutation loci in the predetermined region and possible base types for each mutation locus can be determined by alignment.

At step S220, a number of sequencing reads corresponding to each of the base types is determined.

In order to determine the base type of a mutation locus, the number of sequencing reads corresponding to each base type was determined by counting sequencing reads supporting the base type.

At step S230, the number of all sequencing reads corresponding to the mutation locus is determined.

As previously described, in order to determine the base type of a mutation locus, the number of all sequencing reads corresponding to the mutation locus can be determined by counting sequencing reads supporting the mutation locus.

At step S240, a proportion of sequencing reads for the base type is determined.

For a specific mutation locus, a proportion of sequencing reads for each base type can be determined by determining the sequencing reads corresponding to each base type and the number of sequencing reads corresponding to the mutation locus. For example, for a certain mutation locus, if there are 100 sequencing reads supporting the mutation locus and 50 sequencing reads supporting base A, then the proportion of sequencing reads for base A is 50/100 = 50%.

At step S250, a specific base type of the mutation locus is determined.

According to an embodiment of the present disclosure, the specific base type refers to at least one of a major allele type or a minor allele type. For a certain mutation locus, after determining the proportion of sequencing reads of each base type, the base type with the highest proportion of sequencing reads can be selected as the major allele type, and the base type with the second highest proportion of sequencing reads can be selected as the minor allele type.

At step S260, a frequency of the major allele type or the minor allele type in the corresponding mutation locus is determined.

After the specific base type, i.e., the major allele type or the minor allele type, is determined, the proportion of sequencing reads corresponding to the specific base type is taken as the frequency of the specific base type in the corresponding mutation locus.

According to an embodiment of the present disclosure, for the convenience of calculation, it may be assumed that only a major allele type and a minor allele type are present, and thus a sum of the frequency of the major allele type and the frequency of the minor allele type is 100%.

Specifically, according to an embodiment of the present disclosure, determining the mutation information in the predetermined region includes: (2-1) aligning the sequencing data with the reference genome sequence to determine mutation loci in the predetermined region and a base type for each of the mutation loci; (2-2) for each of the mutation loci, determining a specific base type based on numbers of sequencing reads corresponding to respective base types, and determining a frequency of the specific base type, so as to obtain a plurality of frequencies; and (2-3) determining, based on each of the plurality of frequencies, a proportion of mutation loci corresponding to the frequency, the proportion of the mutation loci characterizing a number of the mutation loci corresponding to the frequency. It should be noted that the proportion of the mutation loci used in step (2-3) can be directly represented by the number of mutation loci corresponding to the frequency, or by any mathematical operation result based on the number of the mutation loci and a total mutation locus number, as long as it can characterize the number of mutation loci corresponding to the same frequency, thereby reflecting the distribution of loci corresponding to the specific base type in the region.

According to an embodiment of the present disclosure, upon determining the major allele type or minor allele type of each mutation locus and the corresponding frequency of occurrence, and acquiring the number or proportion of mutation loci at each identical frequency, the cell-free fetal nucleic acid concentration in the predetermined region can be then determined by the following steps:

(3-1) determining a distribution of the proportion of the mutation loci with respect to the frequency; and
(3-2) determining the cell-free fetal nucleic acid concentration corresponding to the predetermined region based on the distribution.

More specifically, in step (3-1), the proportion of the mutation loci is two-dimensionally plotted against the plurality of frequencies within a predetermined frequency range; and in step (3-2), the cell-free fetal nucleic acid concentration is determined based on frequencies corresponding to peaks and troughs of the two-dimensionally plotted graph.

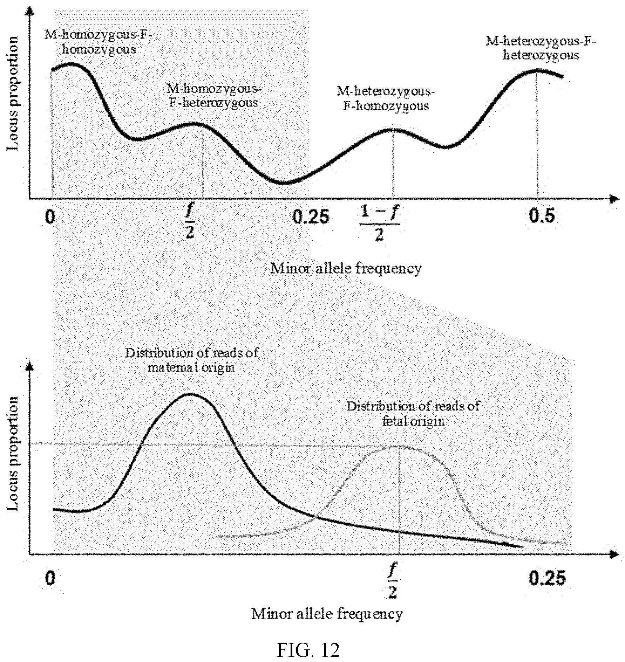

To facilitate understanding, taking the minor allele type as an example, the whole genome is partitioned into different sliding windows with a window size that may be 100 kb, in order to calculate regional fetal fraction using maternal plasma cfDNA data. Within each sliding window, the minor allele frequency (MAF) of each mutation locus is calculated respectively, and the distribution of each minor allele frequency, i.e. the number of mutation loci corresponding to the same minor allele frequency, is calculated. Through researches, the inventors concluded that for the distribution of minor allele frequencies, there could theoretically be four peaks corresponding to four types of loci: maternal-homozygous-fetal-homozygous (M-homozygous-F-homozygous), maternal-homozygous-fetal-heterozygous (M-homozygous-F-heterozygous), maternal-heterozygous-fetal-homozygous (M-heterozygous-F-homozygous), and maternal-heterozygous-fetal-heterozygous (M-heterozygous-F-heterozygous). It is known that the MAF distribution consists of sequencing reads from both the pregnant women and the fetus, so that for each locus, when the fetal fraction is $f$, the MAF corresponding to the peak of the M-homozygous-F-heterozygous locus is $f/2$. Therefore, for MAF $\in$ [0, 0.25], its distribution can be simulated as two hybrid models with a normal distribution, and then it can be inferred that the MAF corresponding to the peak value of the distribution of reads of fetal origin is $f/2$, namely, obtaining the fetal fraction in this window, see FIG. 12. Of course, those skilled in the art will appreciate that similar analysis using the major allele type can be used to analyze fetal fraction.

Thus, according to an embodiment of the present disclosure, the predetermined frequency range is selected from 0 to 0.5 or from 0.5 to 1.

According to an embodiment of the present disclosure, the specific base type is a minor allele type, and the predetermined frequency range is a range from 0 to 0.25 or a subset thereof or a range from 0.25 to 0.5 or a subset thereof. Here, when the predetermined frequency range is a range from 0 to 0.25 or a subset thereof, an allele frequency having a value of a corresponding to a second peak of the peaks in a frequency increasing direction is selected, and the fetal fraction is 2a; and when the predetermined frequency range is a range from 0.25 to 0.5 or a subset thereof, an allele frequency having a value of b corresponding to a first peak of the peaks in a frequency increasing direction is selected, and the fetal fraction is 1-2b.

According to an embodiment of the present disclosure, the specific base type is a major allele type, and the predetermined frequency range is a range from 0.5 to 0.75 or a subset thereof or a range from 0.75 to 1 or a subset thereof. Here, when the predetermined frequency range is a range from 0.5 to 0.75 or a subset thereof, an allele frequency having a value of c corresponding to a second peak of the peaks in a frequency increasing direction is selected, and the fetal fraction is 2c; and when the predetermined frequency range is from 0.75 to 1 or a subset thereof, an allele frequency having a value of d corresponding to a first peak of the peaks in a frequency increasing direction is selected, and the fetal fraction is 1-2d.

Therefore, by the method provided by the embodiment of the present disclosure, the concentration of the cell-free fetal nucleic acids in a specific region can be effectively determined through the base type distribution of the mutation loci, and the proportion of the cell-free fetal nucleic acids in the region to the cell-free maternal nucleic acids in the region can be truly reflected, so that the method can be more effectively used for guiding the fetal gene analysis.

On the basis of this, in a second aspect of the present disclosure, the present disclosure provides a method for determining a fetal genotype at a predetermined locus.

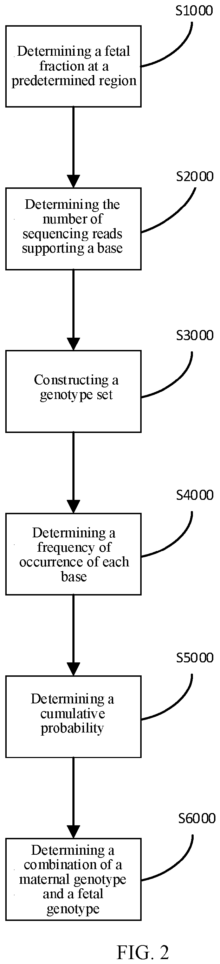

Referring to FIG. 2, according to an embodiment of the present disclosure, the method includes the following steps.

At step S1000, a concentration of cell-free fetal nucleic acids corresponding to a predetermined region including the predetermined locus is determined according to the method described above. The determination of the cell-free fetal nucleic acid concentration in a predetermined region has been described in detail above and will not be repeated here. With respect to the predetermined locus, it may be any locus of interest to the analyst where a mutation may be present, e.g. a locus that is likely to cause disease or may be used to distinguish fetal identity, e.g. for paternity test.

At step S2000, a number $A_j$ (j being A, T, G, or C) of sequencing reads supporting base A, base T, base G, or base C is determined respectively for the predetermined locus;

At step S3000, a genotype set $\{M_{1i}M_{2i}P_{1i}P_{2i}\}$ is constructed for the predetermined locus, where i denotes a genotype serial number, $M_{1i}$ denotes a base type of the predetermined locus on a first maternal chromosome for the i-th genotype, $M_{2i}$ denotes a base type of the predetermined locus on a second maternal chromosome for the i-th genotype, $F_{1i}$ denotes a base type of the predetermined locus on a first fetal chromosome for the i-th genotype, $F_{2i}$ denotes a base type of the predetermined locus on a second fetal chromosome for the i-th genotype, and $M_{1i}$, $M_{2i}$, $F_{1i}$, and $F_{2i}$ are each independently base A, base T, base G, or base C. Here, the first maternal chromosome and the second maternal chromosome constitute a pair of homologous chromosomes, and the first fetal chromosome and the second fetal chromosome constitute a pair of homologous chromosomes.

At step S4000, a probability $P_j$ of occurrence of each base for each genotype of the genotype set $\{M_{1i}M_{2i}P_{1i}P_{2i}\}$ is determined based on the cell-free fetal nucleic acid concentration, where j is A, T, G, or C.

At step S5000, a cumulative probability $P\,(M_{1i}M_{2i}F_{1i}F_{2i})$ of each genotype of the genotype set $\{M_{1i}M_{2i}P_{1i}P_{2i}\}$ is determined based on the probability Pj of occurrence of each base and the number $A_j$ of the sequencing reads.

At step S6000, a combination of a maternal genotype and a fetal genotype at the predetermined locus is determined based on the cumulative probability $P\,(M_{1i}M_{2i}P_{1i}P_{2i})$ of each genotype, thereby obtaining the fetal genotype at the predetermined locus. In this way, the genotype combination of the fetus and the pregnant woman can be determined effectively based on the fetal nucleic acid concentration in the predetermined region, as well as the number of sequencing reads obtained by sequencing. In other words, the haplotype of a specific locus within the region can be determined, i.e., for the specific locus, the type of allele carried on each of the four chromosomes (two homologous chromosomes from mother and two corresponding homologous chromosomes from the fetus) can be determined.

To facilitate understanding, the principles of the above analytical methods are analyzed as follows:
According to an embodiment of the present disclosure, after the fetal fraction is obtained, a Bayesian model is established on this basis to infer the fetal genotype. When the read depth of different alleles in maternal plasma and the fetal fraction are known, the probability of obtaining a maternal and fetal genotype combination i at a specific locus is calculated as:

$$P(A_i|B) = \frac{P(B|A_i)P(A_i)}{\sum_{i=1}^{n} P(B|A_i)P(A_i)},$$

where $P(A_i)$ is a prior probability of occurrence of the combination i among n maternal and fetal genotype combinations obtained based on the fetal fraction; $P(B|A_i)$ is a cumulative probability with the maternal and fetal genotype combination i calculated based on the depth of the sequencing reads at the locus and the prior probability; and the n maternal and fetal genotype combinations = (10 maternal fetal genotypes $\times$ 10 fetal genotypes).

According to an embodiment of the present disclosure, the probability $P_j$ of occurrence is determined based on the following formula:

$$P_j = \frac{B_{jF}}{2}C + \frac{B_{jM}}{2}(1 - C),$$

where $B_{jF}$ is an integer from 0 to 2 and represents a number of occurrences of base j in $F_{1i}F_{2i}$, C represents the fetal nucleic acid concentration at the predetermined region, and $B_{jM}$ is an integer from 0 to 2 and represents a number of occurrences of base j in $M_{1i}M_{2i}$.

According to an embodiment of the disclosure, the cumulative probability P ($M_{1i}M_{2i}F_{1i}F_{2i}$) is determined based on the following formula:

$$P\left(M_{1i}M_{2i}F_{1i}F_{2i}\right) = \frac{\sum P_j A_j}{\sum A_j},$$

where j represents the base occurring in $M_{1i}M_{2i}F_{1i}F_{2i}$.

According to an embodiment of the present disclosure, step (f) further includes determining a final cumulative probability $P_{final}$ ($M_{1i}M_{2i}P_{1i}P_{2i}$) for each genotype, and selecting a genotype with the highest final probability as the combination of the maternal genotype and the fetal genotype at the predetermined locus, thereby obtaining the fetal genotype at the predetermined locus, where the final cumulative probability $P_{final}$ ($M_{1i}M_{2i}P_{1i}P_{2i}$) is determined by the following formula:

$$P_{final}\left(M_{1i}M_{2i}F_{1i}F_{2i}\right) = \frac{P\left(M_{1i}M_{2i}F_{1i}F_{2i}\right)}{\sum P\left(M_{1i}M_{2i}F_{1i}F_{2i}\right)}.$$

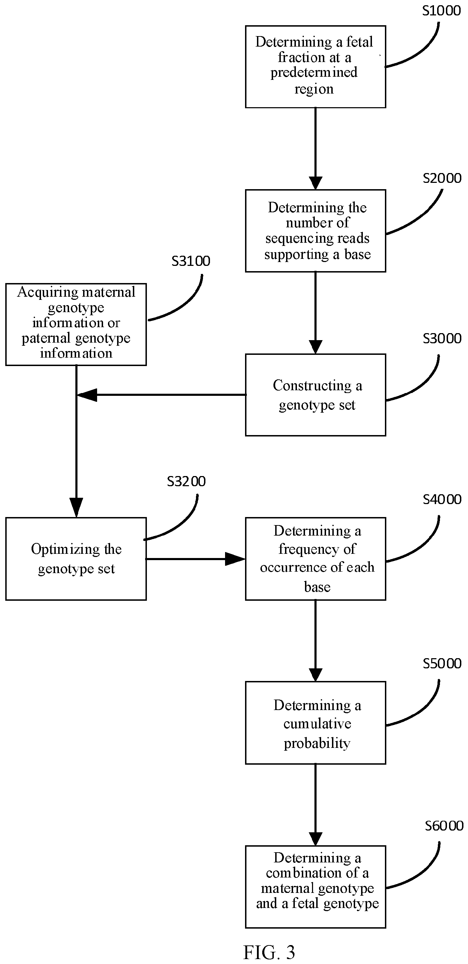

According to an embodiment of the present disclosure, with reference to FIG. 3, the method may further include, subsequent to step S3000:

S3100: acquiring at least one of maternal genotype information or paternal genotype information of the fetus; and
S3200: optimizing the genotype set $\{M_{1i}M_{2i}P_{1i}P_{2i}\}$ based on the at least one of the maternal genotype information or the paternal genotype information.

According to an embodiment of the disclosure, the maternal genotype information and the paternal genotype information are generated by gene sequencing of nucleated cells, and the optimizing includes removing genotypes that do not comply with Mendelian inheritance rules from the genotype set $\{M_{1i}M_{2i}P_{1i}P_{2i}\}$. Thus, by determining the maternal genotype information and the paternal genotype information, and optimizing the genotype set that may be present, the accuracy for determination of the fetal genotype can be improved. In other words, when the maternal and paternal genotypes are known, the combination of prior probabilities can be modified for the above Bayesian model to remove the maternal and fetal genotype combinations that do not comply with Mendelian inheritance rules and the probability of each combination is recalculated. Finally, the combination with the highest probability is selected to obtain the fetal genotype.

According an embodiment of the present disclosure, the maternal genotype information and the paternal genotype information may be obtained by any known means, for example, by sequencing paternal or maternal nucleated cells, and may also be acquired by single-tube long fragment read (stLFR) sequencing, whereby the maternal and paternal haplotypes within the region can be efficiently obtained to more efficiently correct the obtained results. According to an embodiment of the disclosure, a plurality of predetermined loci may be included. After a fetal genotype at one predetermined locus is obtained, the genotypes at more loci may also be analysed, thus obtaining fetal genotype results at more loci. It will be appreciated by those skilled in the art that the determination of the fetal genotypes at a plurality of predetermined loci needs to analyze each locus needs independently, i.e. the method steps described above are performed for each predetermined locus.

According to an embodiment of the present disclosure, after the reference genome sequence is obtained, the reference genome sequence is partitioned into a plurality of regions including the predetermined region, the predetermined region including the predetermined locus.

According to an embodiment of the present disclosure, the reference genome sequence may be partitioned into a plurality of continuous or discrete regions each having a length from 50 kb to 200 kb. Thus, the inclusion of a sufficient number of mutations in each of these regions allows analysis of the concentration of cell-free fetal nucleic acids in the region, while ensuring that the resulting concentration of cell-free fetal nucleic acids truly reflects the distribution or proportion of cell-free fetal nucleic acids in the region, ensuring the accuracy of the genotyping.

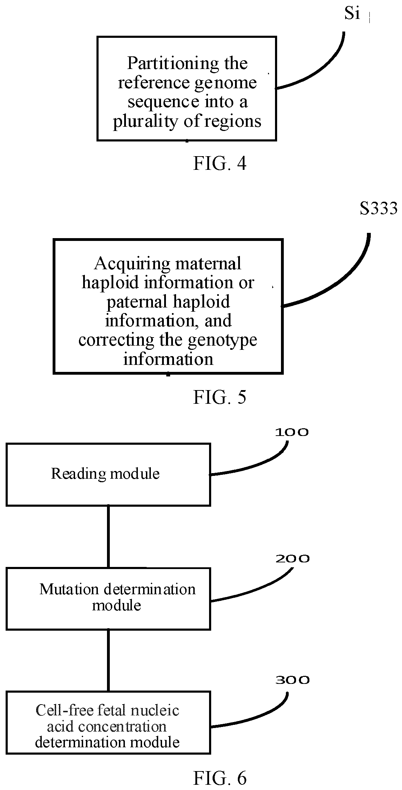

Referring to FIG. 5, according to an embodiment of the present disclosure, the above-described method for genetic analysis of the pregnant women and fetus at predetermined loci may further include the following step subsequent to S6000:

S333: acquiring at least one of maternal haploid information or paternal haploid information of the fetus, and correcting, based on the at least one of the maternal haploid information or the paternal haploid information, the obtained result of the maternal and fetal genotype combination.

According to an embodiment of the disclosure, the correcting is performed based on a linkage disequilibrium. According to an embodiment of the present disclosure, the correcting is performed using a linkage disequilibrium of 200 to 400 loci (preferably 300 loci) flanking the predetermined locus. According to an embodiment of the present disclosure, when too few loci are selected, a problem may occur in the determination of a parent or maternal origin due to insufficient locus information or an inferential error of the original locus per se. On the other hand, when excessive flanking loci are used, additional errors may be introduced due to the increased chromosome recombination rate (the longer the selected genomic region, the greater the probability of recombination will be). In addition, the calculation efficiency also needs to be considered, and the greater the number of the selected flanking loci, the longer the calculation time is.

According to an embodiment of the disclosure, at least one of the maternal haploid information or the paternal haploid information is generated by single-tube long fragment read sequencing of nucleated cells.

Specifically, according to an embodiment of the present disclosure, blood nucleated cells of a pregnant women and her husband are subjected to single-tube long fragment read sequencing, and the resulting sequencing data is subjected to assembly of long fragments and analysis of sample haplotype using tools such as Hapcut2 or Longhap. Of course, if conventional sequencing data is used, haplotype analysis can be accomplished using tools such as SHAPEIT or BEAGLE with only short sequencing reads. When the paternal and maternal haplotype results are known, on the basis of the fetal genotype of the single locus, the linkage disequilibrium (LD) relationship of the confidence loci flanking the locus can be used to correct the loci with low accuracy. In brief, for the fetal locus to be corrected, two haplotypes where two alleles (base types) inherited from father and mother at the locus are located are respectively obtained by inferring, and the allele (base types) actually at the locus in the two haplotypes is extracted as a new fetal genotype. For example, when the paternal haplotype is inferred, a paternal-heterozygous-maternal-homozygous locus is used: when the father is AC, the mother is AA, and the fetus is AC, it is determined that the haplotype where father C allele (base type) is located is inherited to the fetus; when the father is AC, and the fetus is AA, it is determined that the haplotype where father A allele (base type) is located is inherited to the fetus. When the maternal haplotype is inferred, a maternal-heterozygous-fetal homozygous locus is used: when the mother is AC and the fetus is CC, it is determined that the mother C allele is inherited to the fetus. Then, both sides are extended with the analyzed locus as a center to provide a certain number of usable loci, and the haplotype with the highest proportion inferred by these loci (i.e., a genetic haplotype) is calculated. Returning to the results of the paternal and maternal haplotypes, the alleles (base types) of the two haplotypes inherited to the fetus at that locus were found and combined into a new genotype of the fetus.

Currently, most fetal genome analysis based on maternal plasma cfDNA whole genome sequencing data focuses on the detection of chromosomal level structural variation, large fragment copy number mutation, and paternal specific variation. According to an embodiment of the present disclosure, the present disclosure can extend the fetal genome inference range using maternal plasma cfDNA whole genome sequencing data to whole genome wide single locus variation, improve the detection accuracy to mono-allelic mutation, and expand the application range of fetal genetic disease detection. In addition, according to an embodiment of the present disclosure, the present disclosure can also provide various analysis methods (using only pregnant women plasma data, using different types of data such as data of pregnant women plasma and data of blood nucleated cells from the pregnant women and her husband, etc.), which are applicable to different items and different sample types. According to an embodiment of the present disclosure, the present disclosure further improves the accuracy of fetal genotype inference using only maternal plasma by combining local fetal fractions with the Bayesian model. In addition, the fetal genome analysis method currently performed using only maternal plasma cfDNA has very high requirements for sequencing depth (300X or above), and according to the embodiments of the present disclosure, the inventors have surprisingly found that the method of the present disclosure still achieves high accuracy at relatively low sequencing depth (about 60X to 100X).

Corresponding to the method described above, the embodiments of the present application also provide a corresponding apparatus for implementing the method described above.

In particular, in a third aspect, the present disclosure provides a device for determining the concentration of cell-free fetal nucleic acids. Referring to FIG. 6, according to embodiments of the present disclosure, the device includes: a reading module 100 configured to acquire sequencing data of a first nucleic acid sample of a pregnant woman and a reference genome sequence, the first nucleic acid sample of the pregnant woman containing cell-free fetal nucleic acids, and the sequencing data being composed of a plurality of sequencing reads; a mutation determination module 200 configured to select a predetermined region on the reference genome sequence and determining, based on the sequencing data of the first nucleic acid sample of the pregnant woman, mutation information in the predetermined region; and a cell-free fetal nucleic acid concentration determination module 300 configured to determine the concentration of cell-free fetal nucleic acids corresponding to the predetermined region based on the mutation information in the predetermined region.

According to the embodiments of the present disclosure, the device is capable of effectively performing the foregoing method for determining the concentration of cell-free fetal nucleic acids. The advantages and features described with respect to the above method are applicable to the device and will not be described in detail.

According to an embodiment of the present disclosure, the predetermined region is 50 kb to 200 kb in length.

According to an embodiment of the present disclosure, the first nucleic acid sample of the pregnant woman is derived from peripheral blood of the pregnant woman and the mutation information includes at least one of SNP, Indel, or SV.

According to an embodiment of the present disclosure, the mutation determination module 200 is adapted to: align the sequencing data with the reference genome sequence to determine mutation loci in the predetermined region and a base type for each of the mutation loci; for each of the mutation loci, determine a specific base type based on numbers of sequencing reads corresponding to respective base types, and determining a frequency of the specific base type, so as to obtain a plurality of frequencies; and determine, based on each of the plurality of frequencies, a proportion of mutation loci corresponding to the frequency, the proportion of the mutation loci characterizing a number of the mutation loci corresponding to the frequency.

According to an embodiment of the disclosure, the cell-free fetal nucleic acid concentration determination module 300 is adapted to: determine a distribution of the proportion of the mutation loci with respect to the frequency; and determine the cell-free fetal nucleic acid concentration corresponding to the predetermined region based on the distribution.

According to an embodiment of the disclosure, the cell-free fetal nucleic acid concentration determination module 300 is adapted to: two-dimensionally plot the proportion of the mutation loci against the plurality of frequencies within a predetermined frequency range; and determine the cell-free fetal nucleic acid concentration based on frequencies corresponding to peaks and troughs of the two-dimensionally plotted graph.

According to an embodiment of the present disclosure, the predetermined frequency range is selected from 0 to 0.5 or from 0.5 to 1.

According to an embodiment of the present disclosure, the specific base type is a minor allele type, and the predetermined frequency range is a range from 0 to 0.25 or a subset thereof or a range from 0.25 to 0.5 or a subset thereof; when the predetermined frequency range is a range from 0 to 0.25 or a subset thereof, an allele frequency having a value of a corresponding to a second peak of the peaks in a frequency increasing direction is selected, and the fetal fraction is 2a; and when the predetermined frequency range is a range from 0.25 to 0.5 or a subset thereof, an allele frequency having a value of b corresponding to a first peak of the peaks in a frequency increasing direction is selected, and the fetal fraction is 1-2b.

According to an embodiment of the present disclosure, the specific base type is a major allele type, and the predetermined frequency range is a range from 0.5 to 0.75 or a subset thereof or a range from 0.75 to 1 or a subset thereof; when the predetermined frequency range is a range from 0.5 to 0.75 or a subset thereof, an allele frequency having a value of c corresponding to a second peak of the peaks in a frequency increasing direction is selected, and the fetal fraction is 2c; and when the predetermined frequency range is a range from 0.75 to 1 or a subset thereof, an allele frequency having a value of d corresponding to a first peak of the peaks in a frequency increasing direction is selected, and the fetal fraction is 1-2d.

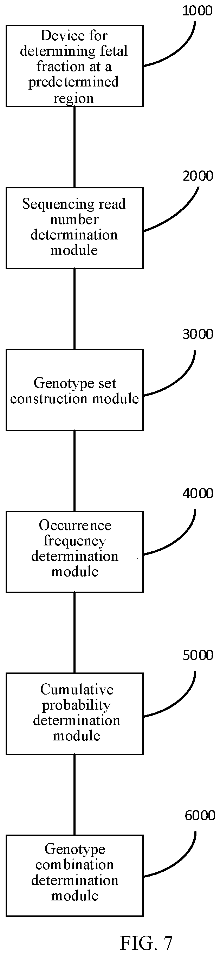

In a fourth aspect, the present disclosure provides an apparatus for determining a fetal genotype at a predetermined locus. Referring to FIG. 7, the apparatus includes:

the device 1000 for determining the concentration of cell-free fetal nucleic acids as mentioned above for determining the concentration of cell-free fetal nucleic acids in the predetermined region, the predetermined region including the predetermined locus;

a sequencing read number determination module 2000 configured to determine a number $A_j$ (j being A, T, G, or C) of sequencing reads supporting base A, base T, base G, or base C, respectively, for the predetermined locus;

a genotype set construction module 3000 configured to constructing a genotype set $\{M_{1i}M_{2i}F_{1i}F_{2i}\}$ for the predetermined locus, wherein i denotes a genotype serial number, $M_{1i}$ denotes a base type of the predetermined locus on a first maternal chromosome for the i-th genotype, $M_{2i}$ denotes a base type of the predetermined locus on a second maternal chromosome for the i-th genotype, $F_{1i}$ denotes a base type of the predetermined locus on a first fetal chromosome for the i-th genotype, $F_{2i}$ denotes a base type of the predetermined locus on a second fetal chromosome for the i-th genotype, and $M_{1i}$, $M_{2i}$, $F_{1i}$, and $F_{2i}$ are each independently base A, base T, base G, or base C, where the first maternal chromosome and the second maternal chromosome constitute a pair of homologous chromosomes, and the first fetal chromosome and the second fetal chromosome constitute a pair of homologous chromosomes;

an occurrence probability determination module 4000 configured to determine a probability $P_j$ of occurrence of each base for each genotype of the genotype set $\{M_{1i}M_{2i}P_{1i}P_{2i}\}$ based on the cell-free fetal nucleic acid concentration, wherein j is A, T, G, or C.

a cumulative probability determination module 5000 configured to determine a cumulative probability $P(M_{1i}M_{2i}P_{1i}P_{2i})$ of each genotype of the genotype set $\{M_{1i}M_{2i}P_{1i}P_{2i}\}$ based on the probability Pj of occurrence of each base and the number $A_j$ of the sequencing reads; and

a genotype combination determination module 6000 configured to determine a combination of a maternal genotype and a fetal genotype at the predetermined locus based on the cumulative probability $P(M_{1i}M_{2i}P_{1i}P_{2i})$ of each genotype, thereby obtaining the fetal genotype at the predetermined locus.

According to an embodiment of the disclosure, the probability $P_j$ of occurrence is determined based on the following formula:

$$P_j = \frac{B_{jF}}{2}C + \frac{B_{jM}}{2}(1 - C),$$

where $B_{jF}$ is an integer from 0 to 2 and represents a number of occurrences of base j in $F_{1i}F_{2i}$, C represents the fetal nucleic acid concentration at the predetermined region, and $B_{jM}$ is an integer from 0 to 2 and represents a number of occurrences of base j in $M_{1i}M_{2i}$.

According to an embodiment of the disclosure, the cumulative probability $P(M_{1i}M_{2i}F_{1i}F_{2i})$ is determined based on the following formula:

$$P\left(M_{1i}M_{2i}F_{1i}F_{2i}\right) = \frac{\sum P_j A_j}{\sum A_j},$$

where j represents the base occurring in $M_{1i}M_{2i}F_{1i}F_{2i}$.

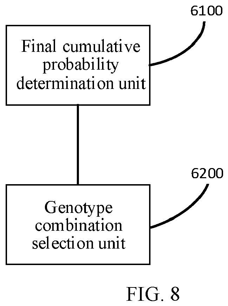

Referring to FIG. 8, according to an embodiment of the present disclosure, the genotype combination determination module 6000 includes: a final cumulative probability determination unit 6100 configured to determine a final cumulative probability $P_{final}(M_{1i}M_{2i}F_{1i}F_{2i})$ for each genotype; and a genotype combination selection unit 6200 configured to select a genotype with a highest final cumulative probability as the combination of the maternal genotype and the fetal genotype at the predetermined locus, thereby obtaining the fetal genotype at the predetermined locus, where the final cumulative probability $P_{final}(M_{1i}M_{2i}F_{1i}F_{2i})$ is determined by the following formula:

$$P_{final}\left(M_{1i}M_{2i}F_{1i}F_{2i}\right) = \frac{P\left(M_{1i}M_{2i}F_{1i}F_{2i}\right)}{\sum P\left(M_{1i}M_{2i}F_{1i}F_{2i}\right)}.$$

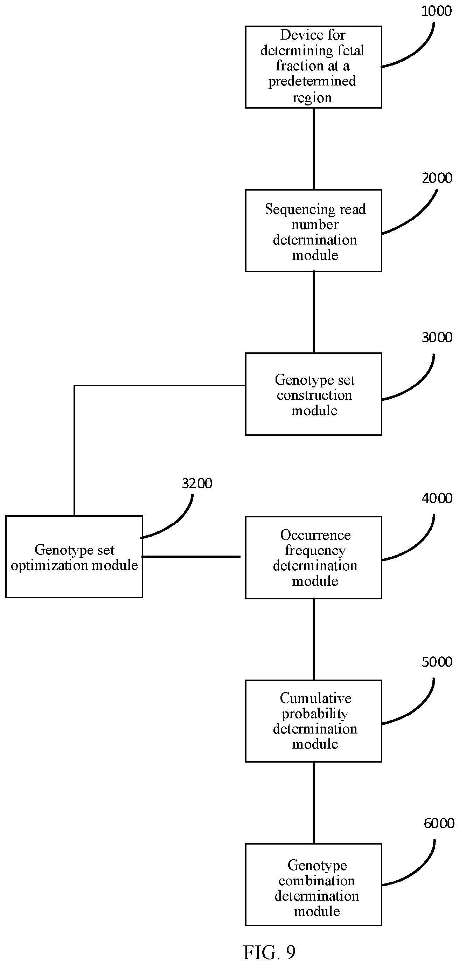

Referring to FIG. 9, according to an embodiment of the present disclosure, a genotype set optimization module 3200 is further included for acquiring at least one of at least one of maternal genotype information or paternal genotype information of the fetus; and optimizing the genotype set $\{M_{1i}M_{2i}P_{1i}P_{2i}\}$ based on the at least one of the maternal genotype information or the paternal genotype information.

According to an embodiment of the disclosure, the maternal genotype information and the paternal genotype information are generated by gene sequencing of nucleated cells, and the optimization process module includes a filtering unit configured to remove genotypes that do not comply with Mendelian inheritance rules from the genotype set $\{M_{1i}M_{2i}P_{1i}P_{2i}\}$.

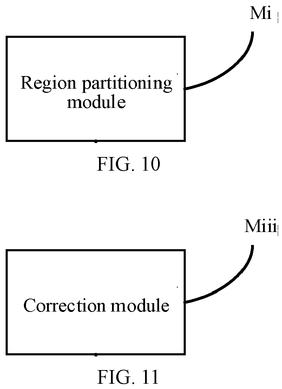

Referring to FIG. 10, according to an embodiment of the present disclosure, a region partitioning module Mi is further included for partitioning the reference genome sequence into a plurality of regions including the predetermined region after the reference genome sequence is acquired, the predetermined region including the predetermined locus.

According to an embodiment of the present disclosure, the predetermined locus includes a plurality of mutation loci.

Referring to FIG. 11, according to an embodiment of the present disclosure, the apparatus for determining the fetal genotype at the predetermined locus further includes:

a correction module Miii configured to acquire at least one of maternal haploid information or paternal haploid information of the fetus, and correct, based on the at least one of the maternal haploid information or the paternal haploid information, the combination of the maternal genotype and the fetal genotype.

According to an embodiment of the disclosure, the correcting is performed based on a linkage disequilibrium. According to an embodiment of the present disclosure, the correcting is performed using a linkage disequilibrium of 200 to 400 loci flanking the predetermined locus. Preferably, the correcting is performed using a linkage disequilibrium of 300 loci flanking the predetermined locus.

According to an embodiment of the disclosure, the at least one of the maternal haploid information or the paternal haploid information is generated by single-tube long fragment read sequencing of nucleated cells.

In a fifth aspect of the present disclosure, a computer-readable storage medium is provided, the computer-readable storage medium has a computer program stored thereon, and the program, when executed by a processor, implements the steps of the method described above.

It will be appreciated by those skilled in the art that the features and advantages described in various aspects are equally applicable to other aspects and are not described in detail herein.

The technical solutions of the present disclosure will be explained below with reference to examples. Those skilled in the art will understand that these examples are illustrative only, and should not be considered as limiting the scope of the present disclosure. Examples, where specific techniques or conditions are not specified, are implemented in accordance with techniques or conditions described in the literature in the art or according to the product specification. Examples, where specific conditions are not specified, are implemented in accordance with conventional conditions or those recommended by the manufacturer. All of the used agents or instruments which are not specified with the manufacturer are conventional commercially-available products.

Example 1

Experimental method

Three families were selected to obtain the stLFR sequencing data of maternal plasma cfDNA (cell-free nucleic acids) (whole genome sequencing WGS, paired-end 100 bp sequencing, average depth about 100X) and parental and maternal nucleated blood cells (whole genome sequencing WGS, pair-end 100 bp sequencing, average depth about 40 to 60X), and fetal cord blood whole genome sequencing data (paired-end 100 bp sequencing, average depth about 40X) was used as a true set to calculate the accuracy of fetal genotype inference. The overall accuracy of all loci in each of the 3 families was 95% or above (i.e., regardless of heterozygosity and homozygosity, all subsequent methods can achieve an overall accuracy of 95% or above).

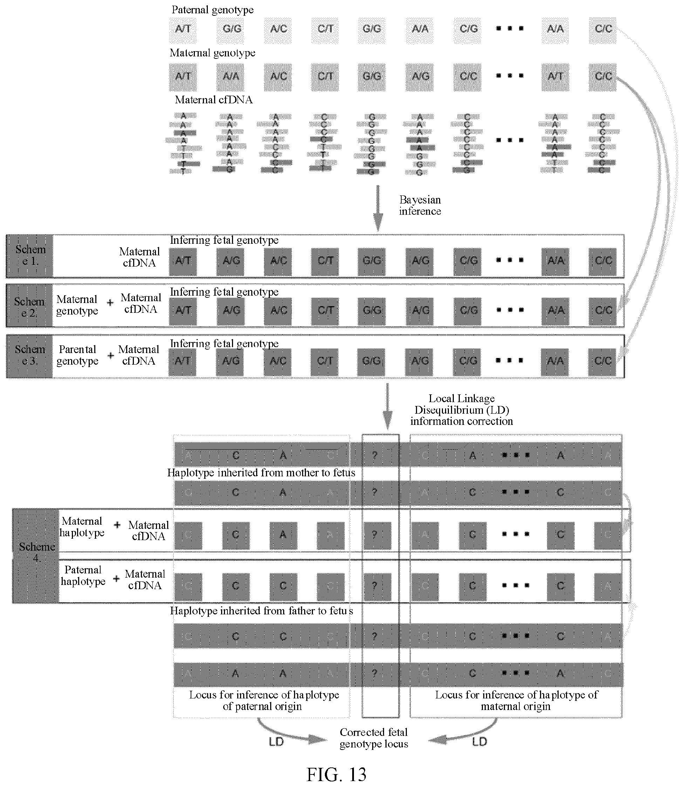

Referring to FIG. 13, the three families described above can be analyzed separately using the following scheme:

Scheme 1: after regional fetal fraction are determined by using only maternal plasma cfDNA (cell-free DNA) sequencing data, the fetal genotype is obtained by Bayesian inference.

Scheme 2 (not directly applied): based on Scheme 1, the fetal genotype of Scheme 1 was optimized using genotype data from stLFR sequencing of maternal nucleated blood cells.

Scheme 3: based on Scheme 1, the fetal genotype of Scheme 1 was optimized using genotype data from stLFR sequencing of maternal and paternal nucleated blood cells.

Scheme 4: based on Scheme 1, the fetal genotype of Scheme 1 was optimized using haplotype data from stLFR sequencing of maternal and paternal nucleated blood cells.

Experimental Results

Based on the maternal and paternal stLFR data of each family, the maternal haplotype and the paternal haplotype were obtained respectively, and each subjected to calculation for the switch error rate which was used to evaluate the accuracy of the stLFR data.

Based on the data of the above three families, the tests of the above Scheme 1 and Scheme 3 were completed respectively to obtain the accuracy of different types of loci, which was summarized in the following table.

Table 1. Summary of accuracy of fetal genome inference at different types of loci by different schemes

| | | All Loci | | M-homozygous-F-homozygous | | M-homozygous-F-heterozygous | | M-heterozygous-F-homozygous | | M-heterozygous-F-heterozygous | |
|---|---|---|---|---|---|---|---|---|---|---|---|
| | | Total | Accuracy | Total | Accuracy | Total | Accuracy | Total | Accuracy | Total | Accuracy |
| Family 1 | Scheme 1 | 982471 | 0.9510 | 941398 | 0.9662 | 13873 | 0.8600 | 14985 | 0.4640 | 12215 | 0.4800 |
| | Scheme 3 | 982471 | 0.9700 | 941398 | 0.9826 | 13873 | 0.9510 | 14985 | 0.5110 | 12215 | 0.5830 |
| Family 2 | Scheme 1 | 1400919 | 0.9510 | 1356067 | 0.9624 | 8455 | 0.8610 | 15147 | 0.4390 | 21250 | 0.6270 |
| | Scheme 3 | 1400919 | 0.9760 | 1356067 | 0.9852 | 8455 | 0.9220 | 15147 | 0.5680 | 21250 | 0.6990 |
| Family 3 | Scheme 1 | 1810021 | 0.9850 | 1764566 | 0.9886 | 14641 | 0.9470 | 15714 | 0.7500 | 15100 | 0.8500 |
| | Scheme 3 | 1810021 | 0.9870 | 1764566 | 0.9898 | 14641 | 0.9630 | 15714 | 0.8410 | 15100 | 0.8340 |

Note: accuracy refers to the accuracy of fetal genotype inference. The calculation method for accuracy is to directly compare the fetal genotype inferred at each locus with the true fetal genotype obtained from fetal cord blood sequencing data to determine if they are consistent, and the accuracy is a proportion of loci with consistent genotypes to all analyzed loci.

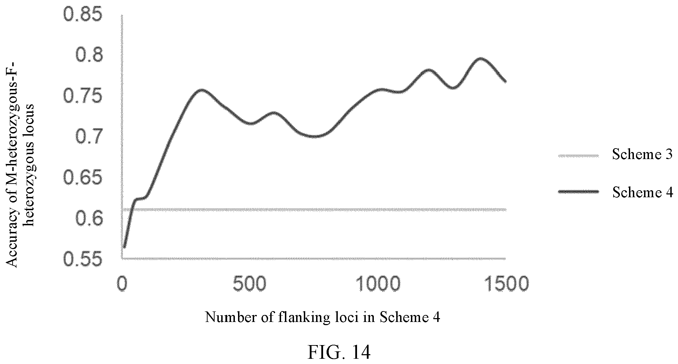

The fetal genotype correction scheme of the Scheme 4 was adopted for the family 1, and the accuracy of the M-heterozygous-F-heterozygous loci with lower accuracy in the Schemes 1 and 3 can be further improved by about 15%; as shown in FIG. 14, the accuracy of the M-heterozygous-F-heterozygous loci in the family 1 corrected by setting different numbers of flanking loci in the scheme 4 was compared with that in the scheme 3, showing that the correction effect is significantly affected by the number of the flanking loci. Thus, 200 to 400 flanking loci, preferably 300 flanking loci, can be adopted for comprehensive consideration.

In addition, reference to the term "an embodiment", "some embodiments", "an example", "a specific example" or "some examples" or the like means that a specific feature, structure, material or characteristic described in combination with the embodiment(s) or example(s) are included in at least one embodiment or example of the present disclosure. In the specification, the schematic description of the above terms is unnecessarily directed to the same example or example. Moreover, the particular features, structures, materials or characteristics described may be combined in any suitable manner in any one or more embodiments or examples.

Although the embodiments of the present disclosure have been illustrated and described, it will be understood by those of ordinary skill in the art that various changes, modifications, replacements and variations can be made to the above embodiments without departing from the principle and ideas of the present disclosure, and the scope of the present disclosure is limited by the appended claims and their equivalents.

## Claims

1. A method for determining a concentration of cell-free fetal nucleic acids, comprising:

   (1) acquiring sequencing data of a first nucleic acid sample of a pregnant woman and a reference genome sequence, the first nucleic acid sample of the pregnant woman containing cell-free fetal nucleic acids, and the sequencing data being composed of a plurality of sequencing reads;
   (2) selecting a predetermined region on the reference genome sequence, and determining, based on the sequencing data of the first nucleic acid sample of the pregnant woman, mutation information in the predetermined region; and
   (3) determining the concentration of cell-free fetal nucleic acids corresponding to the predetermined region based on the mutation information in the predetermined region.

2. The method according to claim 1, wherein the predetermined region is greater than or equal to 50 kb in length.

3. The method according to claim 1, wherein the predetermined region is 50 to 200 kb, preferably 100 kb in length.

4. The method according to claim 1, wherein a sequencing depth of the sequencing data is smaller than or equal to 100X, preferably 60X to 100X.

5. The method according to claim 1, wherein the first nucleic acid sample of the pregnant woman is derived from peripheral blood of the pregnant woman and the mutation information comprises at least one of SNP, Indel, or SV.

6. The method according to claim 1, wherein in step (2), said determining the mutation information in the predetermined region comprises:

   (2-1) aligning the sequencing data with the reference genome sequence to determine mutation loci in the predetermined region and base types for each of the mutation loci;
   (2-2) for each of the mutation loci, determining a specific base type based on numbers of sequencing reads corresponding to respective base types, and determining a frequency of the specific base type, so as to obtain a plurality of frequencies; and
   (2-3) determining, based on each of the plurality of frequencies, a proportion of mutation loci corresponding to the frequency, the proportion of the mutation loci characterizing a number of the mutation loci corresponding to the frequency.

7. The method according to claim 6, wherein step (3) further comprises:

(3-1) determining a distribution of the proportion of the mutation loci with respect to the frequency; and
(3-2) determining the concentration of cell-free fetal nucleic acids corresponding to the predetermined region based on the distribution.

8. The method according to claim 6, further comprising:

in step (3-1), two-dimensionally plotting the proportion of the mutation loci against the plurality of frequencies within a predetermined frequency range; and
in step (3-2), determining the concentration of cell-free fetal nucleic acids based on frequencies corresponding to peaks and troughs of the two-dimensionally plotted graph.

9. The method according to claim 8, wherein the predetermined frequency range is selected from 0 to 0.5 or from 0.5 to 1.

10. The method according to claim 8 or 9, wherein the specific base type is a minor allele type, and the predetermined frequency range is a range from 0 to 0.25 or a subset thereof, or a range from 0.25 to 0.5 or a subset thereof, wherein

when the predetermined frequency range is a range from 0 to 0.25 or a subset thereof, an allele frequency having a value of a corresponding to a second peak of the peaks in a frequency increasing direction is selected, and the concentration of cell-free fetal nucleic acids is 2a; and
when the predetermined frequency range is a range from 0.25 to 0.5 or a subset thereof, an allele frequency having a value of b corresponding to a first peak of the peaks in a frequency increasing direction is selected, and the concentration of cell-free fetal nucleic acids is 1-2b.

11. The method according to any one of claims 8 to 10, wherein the specific base type is a major allele type, and the predetermined frequency range is a range from 0.5 to 0.75 or a subset thereof, or a range from 0.75 to 1 or a subset thereof, wherein

when the predetermined frequency range is a range from 0.5 to 0.75 or a subset thereof, an allele frequency having a value of c corresponding to a second peak of the peaks in a frequency increasing direction is selected, and the concentration of cell-free fetal nucleic acids is 2c; and
when the predetermined frequency range is a range from 0.75 to 1 or a subset thereof, an allele frequency having a value of d corresponding to a first peak of the peaks in a frequency increasing direction is selected, and the concentration of cell-free fetal nucleic acids is 1-2d.

12. A method for determining a fetal genotype at a predetermined locus, comprising:

(a) determining a concentration of cell-free fetal nucleic acids corresponding to a predetermined region comprising the predetermined locus, according to the method of any one of claims 1 to 11;
(b) determining a number $A_j$ (j being A, T, G, or C) of sequencing reads supporting base A, base T, base G, or base C, respectively, for the predetermined locus;
(c) constructing a genotype set $\{M_{1i}M_{2i}P_{1i}P_{2i}\}$ for the predetermined locus, wherein i denotes a genotype serial number, $M_{1i}$ denotes a base type of the predetermined locus on a first maternal chromosome for the i-th genotype, $M_{2i}$ denotes a base type of the predetermined locus on a second maternal chromosome for the i-th genotype, $F_{1i}$ denotes a base type of the predetermined locus on a first fetal chromosome for the i-th genotype, $F_{2i}$ denotes a base type of the predetermined locus on a second fetal chromosome for the i-th genotype, and $M_{1i}$, $M_{2i}$, $F_{1i}$, and $F_{2i}$ are each independently base A, base T, base G, or base C, wherein the first maternal chromosome and the second maternal chromosome constitute a pair of homologous chromosomes, and the first fetal chromosome and the second fetal chromosome constitute a pair of homologous chromosomes;
(d) determining a probability $P_j$ of occurrence of each base for each genotype of the genotype set $\{M_{1i}M_{2i}P_{1i}P_{2i}\}$ based on the concentration of cell-free fetal nucleic acids, wherein j is A, T, G, or C;
(e) determining a cumulative probability P $(M_{1i}M_{2i}P_{1i}P_{2i})$ of each genotype of the genotype set $\{M_{1i}M_{2i}F_{1i}F_{2i}\}$ based on the probability Pj of occurrence of each base and the number $A_j$ of the sequencing reads; and
(f) determining a combination of a maternal genotype and a fetal genotype at the predetermined locus based on the cumulative probability P $(M_{1i}M_{2i}F_{1i}F_{2i})$ of each genotype, thereby obtaining the fetal genotype at the predetermined locus.

13. The method according to claim 12, wherein the probability $P_j$ of occurrence is determined based on the following formula:

$$P_j = \frac{B_{jF}}{2} C + \frac{B_{jM}}{2}(1 - C),$$

where $B_{jF}$ is an integer from 0 to 2 and represents a number of occurrences of base j in $F_{1i}F_{2i}$, C represents the concentration of cell-free fetal nucleic acids corresponding to the predetermined region; and $B_{jM}$ is an integer from 0 to 2 and represents a number of occurrences of base j in $M_{1i}M_{2i}$.

**14.** The method according to claim 12, wherein the cumulative probability P ($M_{1i}M_{2i}F_{1i}F_{2i}$) is determined based on the following formula:

$$P\!\left(M_{1i}M_{2i}F_{1i}F_{2i}\right) = \frac{\sum P_j A_j}{\sum A_j},$$

where j represents the base occurring in $M_{1i}M_{2i}F_{1i}F_{2i}$.

**15.** The method according to claim 12, wherein step (f) further comprises determining a final cumulative probability $P_{final}$ ($M_{1i}M_{2i}P_{1i}P_{2i}$) for each genotype, and selecting a genotype with a highest final cumulative probability as the combination of the maternal genotype and the fetal genotype at the predetermined locus, thereby obtaining the fetal genotype at the predetermined locus, wherein the final cumulative probability $P_{final}$ ($M_{1i}M_{2i}P_{1i}P_{2i}$) is determined by the following formula:

$$P_{final}\;\left(M_{1i}M_{2i}F_{1i}F_{2i}\right) = \frac{P\!\left(M_{1i}M_{2i}F_{1i}F_{2i}\right)}{\sum P\!\left(M_{1i}M_{2i}F_{1i}F_{2i}\right)}.$$

**16.** The method according to claim 12, further comprising, subsequent to step (c):

(c-1) acquiring at least one of maternal genotype information or paternal genotype information of the fetus; and
(c-2) optimizing the genotype set {$M_{1i}M_{2i}P_{1i}P_{2i}$} based on the at least one of the maternal genotype information or the paternal genotype information.

**17.** The method according to claim 16, wherein the maternal genotype information and the paternal genotype information are generated by gene sequencing of nucleated cells, and
wherein the optimizing comprises removing genotypes that do not comply with Mendelian inheritance rules from the genotype set {$M_{1i}M_{2i}P_{1i}P_{2i}$}.

**18.** The method according to claim 12, further comprising:
subsequent to acquiring the reference genome sequence, partitioning the reference genome sequence into a plurality of regions comprising the predetermined region, the predetermined region comprising the predetermined locus.

**19.** The method according to claim 12, wherein the predetermined locus comprises a plurality of predetermined loci.

**20.** The method according to claim 12, further comprising:
(g) acquiring at least one of maternal haploid information or paternal haploid information of the fetus, and correcting, based on the at least one of the maternal haploid information or the paternal haploid information, the maternal genotype and the fetal genotype obtained in step (f).

**21.** The method according to claim 20, wherein the correcting is performed based on a linkage disequilibrium.

**22.** The method according to claim 21, wherein the correcting is performed using a linkage disequilibrium of 200 to 400 loci flanking the predetermined locus.

**23.** The method according to claim 20, wherein the at least one of the maternal haploid information or the paternal haploid information is generated by single-tube long fragment read sequencing of nucleated cells.

24. A device for determining a concentration of cell-free fetal nucleic acids, comprising:

a reading module configured to acquire sequencing data of a first nucleic acid sample of a pregnant woman and a reference genome sequence, the first nucleic acid sample of the pregnant woman containing cell-free fetal nucleic acids of a fetus, and the sequencing data being composed of a plurality of sequencing reads;

a mutation determination module configured to select a predetermined region on the reference genome sequence and determining, based on the sequencing data of the first nucleic acid sample of the pregnant woman, mutation information in the predetermined region; and

a cell-free fetal nucleic acid concentration determination module configured to determine the concentration of cell-free fetal nucleic acids corresponding to the predetermined region based on the mutation information in the predetermined region.

25. The device according to claim 24, wherein the predetermined region is 50 to 200 kb, preferably 100 kb in length, or a sequencing depth of the sequencing data is smaller than or equal to 100X, preferably $60\times$ to 100X.

26. The device according to claim 24, wherein the first nucleic acid sample of the pregnant woman is derived from peripheral blood of the pregnant woman and the mutation information comprises at least one of SNP, Indel, or SV.

27. The device according to claim 24, wherein the mutation determination module is adapted to:

align the sequencing data with the reference genome sequence to determine mutation loci in the predetermined region and a base type for each of the mutation loci;

for each of the mutation loci, determine a specific base type based on numbers of sequencing reads corresponding to respective base types, and determining a frequency of the specific base type, so as to obtain a plurality of frequencies; and

determine, based on each of the plurality of frequencies, a proportion of mutation loci corresponding to the frequency, the proportion of the mutation loci characterizing a number of the mutation loci corresponding to the frequency.

28. The device according to claim 27, wherein the cell-free fetal nucleic acid concentration determination module is adapted to:

determine a distribution of the proportion of the mutation loci with respect to the frequency; and

determine the concentration of cell-free fetal nucleic acids corresponding to the predetermined region based on the distribution.

29. The device according to claim 28, wherein the cell-free fetal nucleic acid concentration determination module is adapted to:

two-dimensionally plot the proportion of the mutation loci against the plurality of frequencies within a predetermined frequency range; and

determine the concentration of cell-free fetal nucleic acids based on frequencies corresponding to peaks and troughs of the two-dimensionally plotted graph.

30. The device according to claim 29, wherein the predetermined frequency range is selected from 0 to 0.5 or from 0.5 to 1.

31. The device according to claim 29 or 30, wherein the specific base type is a minor allele type, and the predetermined frequency range is a range from 0 to 0.25 or a subset thereof or a range from 0.25 to 0.5 or a subset thereof, wherein

when the predetermined frequency range is from 0 to 0.25 or a subset thereof, an allele frequency having a value of a corresponding to a second peak of the peaks in a frequency increasing direction is selected, and the concentration of cell-free fetal nucleic acids is 2a; and

when the predetermined frequency range is from 0.25 to 0.5 or a subset thereof, an allele frequency having a value of b corresponding to a first peak of the peaks in a frequency increasing direction is selected, and the concentration of cell-free fetal nucleic acids is 1-2b.

**32.** The device according to any one of claims 29 to 30, wherein the specific base type is a major allele type, and the predetermined frequency range is a range from 0.5 to 0.75 or a subset thereof or a range from 0.75 to 1 or a subset thereof, wherein

when the predetermined frequency range is a range from 0.5 to 0.75 or a subset thereof, an allele frequency having a value of c corresponding to a second peak of the peaks in a frequency increasing direction is selected, and the concentration of cell-free fetal nucleic acids is 2c; and

when the predetermined frequency range is a range from 0.75 to 1 or a subset thereof, an allele frequency having a value of d corresponding to a first peak of the peaks in a frequency increasing direction is selected, and the concentration of cell-free fetal nucleic acids is 1-2d.

**33.** An apparatus for determining a fetal genotype at a predetermined locus, comprising:

the device for determining the concentration of cell-free fetal nucleic acids according to any one of claims 24 to 32 for determining the concentration of cell-free fetal nucleic acids in the predetermined region, the predetermined region comprising the predetermined locus;

a sequencing read number determination module configured to determine a number $A_j$ (j being A, T, G, or C) of sequencing reads supporting base A, base T, base G, or base C, respectively, for the predetermined locus;

a genotype set construction module configured to construct a genotype set $\{M_{1i}M_{2i}P_{1i}P_{2i}\}$ for the predetermined locus, wherein i denotes a genotype serial number, $M_{1i}$ denotes a base type of the predetermined locus on a first maternal chromosome for the i-th genotype, $M_{2i}$ denotes a base type of the predetermined locus on a second maternal chromosome for the i-th genotype, $F_{1i}$ denotes a base type of the predetermined locus on a first fetal chromosome for the i-th genotype, $F_{2i}$ denotes a base type of the predetermined locus on a second fetal chromosome for the i-th genotype, and $M_{1i}$, $M_{2i}$, $F_{1i}$, and $F_{2i}$ are each independently base A, base T, base G, or base C, wherein the first maternal chromosome and the second maternal chromosome constitute a pair of homologous chromosomes, and the first fetal chromosome and the second fetal chromosome constitute a pair of homologous chromosomes;

an occurrence probability determination module configured to determine a probability $P_j$ of occurrence of each base for each genotype of the genotype set $\{M_{1i}M_{2i}P_{1i}P_{2i}\}$ based on the concentration of cell-free fetal nucleic acids, wherein j is A, T, G, or C;

a cumulative probability determination module configured to determine a cumulative probability $P(M_{1i}M_{2i}P_{1i}P_{2i})$ of each genotype of the genotype set $\{M_{1i}M_{2i}P_{1i}P_{2i}\}$ based on the probability Pj of occurrence of each base and the number $A_j$ of the sequencing reads; and

a genotype combination determination module configured to determine a combination of a maternal genotype and a fetal genotype at the predetermined locus based on the cumulative probability $P(M_{1i}M_{2i}P_{1i}P_{2i})$ of each genotype, thereby obtaining the fetal genotype at the predetermined locus.

**34.** The apparatus according to claim 33, wherein the probability $P_j$ of occurrence is determined based on the following formula:

$$P_j = \frac{B_{jF}}{2}C + \frac{B_{jM}}{2}(1 - C)$$

where $B_{jF}$ is an integer from 0 to 2 and represents a number of occurrences of base j in $F_{1i}F_{2i}$, C represents the concentration of cell-free fetal nucleic acids corresponding to the predetermined region; and $B_{jM}$ is an integer from 0 to 2 and represents a number of occurrences of base j in $M_{1i}M_{2i}$.

**35.** The apparatus according to claim 33, wherein the cumulative probability $P(M_{1i}M_{2i}F_{1i}F_{2i})$ is determined based on the following formula:

$$P\left(M_{1i}M_{2i}F_{1i}F_{2i}\right) = \frac{\sum P_j A_j}{\sum A_j}$$

where j represents the base occurring in $M_{1i}M_{2i}F_{1i}F_{2i}$.

**36.** The apparatus according to claim 33, wherein the genotype combination determination module comprises:

a final cumulative probability determination unit configured to determine a final cumulative probability $P_{final}$ ($M_{1i}M_{2i}P_{1i}P_{2i}$) for each genotype; and
a genotype combination selection unit configured to select a genotype with a highest final cumulative probability as the combination of the maternal genotype and the fetal genotype at the predetermined locus, thereby obtaining the fetal genotype at the predetermined locus,
wherein the final cumulative probability $P_{final}$ ($M_{1i}M_{2i}F_{1i}F_{2i}$) is determined by the following formula:

$$P_{final}\ (M_{1i}M_{2i}F_{1i}F_{2i}) = \frac{P(M_{1i}M_{2i}F_{1i}F_{2i})}{\sum P(M_{1i}M_{2i}F_{1i}F_{2i})}$$

**37.** The apparatus according to claim 33, further comprising a genotype set optimization module configured to:

acquire at least one of maternal genotype information or paternal genotype information of the fetus; and
optimize the genotype set $\{M_{1i}M_{2i}P_{1i}P_{2i}\}$ based on the at least one of the maternal genotype information or the paternal genotype information.

**38.** The apparatus according to claim 37, wherein the maternal genotype information and the paternal genotype information are generated by gene sequencing of nucleated cells, and
wherein the genotype set optimization module comprises a filtering unit configured to remove genotypes that do not comply with Mendelian inheritance rules from the genotype set $\{M_{1i}M_{2i}F_{1i}F_{2i}\}$.

**39.** The apparatus according to claim 33, further comprising:
a region partitioning module configured to partition the reference genome sequence into a plurality of regions comprising the predetermined region after the reference genome sequence is acquired, the predetermined region comprising the predetermined locus.

**40.** The apparatus according to claim 39, wherein the predetermined locus comprises a plurality of predetermined loci.

**41.** The apparatus according to claim 33, further comprising:
a correction module configured to acquire at least one of maternal haploid information or paternal haploid information of the fetus, and correct, based on the at least one of the maternal haploid information or the paternal haploid information, the combination of the maternal genotype and the fetal genotype.

**42.** The apparatus according to claim 41, wherein the correcting is performed based on a linkage disequilibrium.

**43.** The device according to claim 42, wherein the correcting is performed using a linkage disequilibrium of 200 to 400 loci flanking the predetermined locus.

**44.** The apparatus according to claim 41, wherein the at least one of the maternal haploid information or the paternal haploid information is generated by single-tube long fragment read sequencing of nucleated cells.

**45.** A computer-readable storage medium having a computer program stored thereon, wherein the program, when executed by a processor, implements steps of the method according to any one of claims 1 to 23.

S100

Acquiring
sequencing data and
reference genome
sequence

S200

Selecting a
predetermined region
and determining
mutation information in
the predetermined region

S300

Determining
concentration of Cell-
free fetal nucleic acids
based on the mutation
information

FIG. 1

S1000

Determining a fetal
fraction at a
predetermined region

S2000

Determining the
number of
sequencing reads
supporting a base

S3000

Constructing a
genotype set

S4000

Determining a
frequency of
occurrence of each
base

S5000

Determining a
cumulative
probability

S6000

Determining a
combination of a
maternal genotype
and a fetal
genotype

FIG. 2

FIG. 3

Si

Partitioning the reference genome sequence into a plurality of regions

FIG. 4

S333

Acquiring maternal haploid information or paternal haploid information, and correcting the genotype information

FIG. 5

100

Reading module

200

Mutation determination module

300

Cell-free fetal nucleic acid concentration determination module

FIG. 6

1000

Device for
determining fetal
fraction at a
predetermined
region

2000

Sequencing read
number
determination
module

3000

Genotype set
construction module

4000

Occurrence
frequency
determination
module

5000

Cumulative
probability
determination
module

6000

Genotype
combination
determination
module

FIG. 7

6100

Final cumulative
probability
determination unit

6200

Genotype
combination
selection unit

FIG. 8

Device for
determining fetal
fraction at a
predetermined
region

1000

Sequencing read
number
determination
module

2000

Genotype set
construction
module

3000

Genotype set
optimization module

3200

Occurrence
frequency
determination
module

4000

Cumulative
probability
determination
module

5000

Genotype
combination
determination
module

6000

FIG. 9

Mi

Region partitioning
module

FIG. 10

Miii

Correction module

FIG. 11

FIG. 12

FIG. 13

FIG. 14

## INTERNATIONAL SEARCH REPORT

| International application No. |
| --- |
| **PCT/CN2020/072841** |

**A. CLASSIFICATION OF SUBJECT MATTER**

C12Q 1/68(2018.01)i; C12M 1/00(2006.01)i

According to International Patent Classification (IPC) or to both national classification and IPC

**B. FIELDS SEARCHED**

Minimum documentation searched (classification system followed by classification symbols)

C12Q; C12M

Documentation searched other than minimum documentation to the extent that such documents are included in the fields searched

Electronic data base consulted during the international search (name of data base and, where practicable, search terms used)

DWPI, SIPOABS, CNABS, CNKI, ISI Web of knowledge, NCBI, Google Scholar: 胎儿, 母体, 游离, 核酸, 测序, 参考, 参照, 突变, 浓度, 含量, DNA, Fetus, maternal, free, nucleic acid, sequencing, reference, mutation, concentration, content, SNP, Indel

**C. DOCUMENTS CONSIDERED TO BE RELEVANT**

| Category* | Citation of document, with indication, where appropriate, of the relevant passages | Relevant to claim No. |
| --- | --- | --- |
| X | CN 104232777 A (TIANJIN BGI TECHNOLOGY CO., LTD.; SHENZHEN BGI DIAGNOSIS CO., LTD.) 24 December 2014 (2014-12-24) claims, and description, paragraph [0017] | 1-5, 24-26 |
| A | CN 104232777 A (TIANJIN BGI TECHNOLOGY CO., LTD.; SHENZHEN BGI DIAGNOSIS CO., LTD.) 24 December 2014 (2014-12-24) entire document | 6-23, 27-44 |
| A | CN 106537401 A (SK TELECOM CO., LTD.) 22 March 2017 (2017-03-22) entire document | 1-44 |

☐ Further documents are listed in the continuation of Box C.    ☑ See patent family annex.

| * Special categories of cited documents: | "T" | later document published after the international filing date or priority date and not in conflict with the application but cited to understand the principle or theory underlying the invention |
| --- | --- | --- |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
| --- | --- |
| **21 February 2020** | **29 July 2021** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/072841**

Box No. II        Observations where certain claims were found unsearchable (Continuation of item 2 of first sheet)

This international search report has not been established in respect of certain claims under Article 17(2)(a) for the following reasons:

1. ☑ Claims Nos.: **45**
   because they relate to subject matter not required to be searched by this Authority, namely:

   [1]   The subject matter of claim 45 is a simple information expression recorded on a carrier, and therefore
         does not warrant an international search according to the criteria set out in PCT Rule 39.1(iv).

2. ☐ Claims Nos.:
   because they relate to parts of the international application that do not comply with the prescribed requirements to such an
   extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2020/072841**

| Patent document cited in search report | | | Publication date (day/month/year) | Patent family member(s) | | | Publication date (day/month/year) |
|---|---|---|---|---|---|---|---|
| CN | 104232777 | A | 24 December 2014 | HK | 1201886 | A1 | 11 September 2015 |
| | | | | CN | 104232777 | B | 24 August 2016 |
| CN | 106537401 | A | 22 March 2017 | US | 2017198348 | A1 | 13 July 2017 |
| | | | | KR | 20160020400 | A | 23 February 2016 |
| | | | | KR | 20160010277 | A | 27 January 2016 |
| | | | | EP | 3171288 | A4 | 11 July 2018 |
| | | | | EP | 3171288 | A1 | 24 May 2017 |
| | | | | KR | 101801871 | B1 | 27 November 2017 |

Form PCT/ISA/210 (patent family annex) (January 2015)